## Europäisches Patentamt
## European Patent Office
## Office européen des brevets

(19)

(11) Publication number : **0 206 277**
**B1**

(12) # EUROPEAN PATENT SPECIFICATION

(45) Date of publication of patent specification :
30.11.88

(51) Int. Cl.⁴ : **C 12 Q   1/04, C 12 Q   1/08**

(21) Application number : **86108418.4**

(22) Date of filing : **20.06.86**

(54) **Medium composition for the identification of microorganisms.**

(30) Priority : **28.06.85 JP 140619/85**

(43) Date of publication of application :
**30.12.86 Bulletin 86/52**

(45) Publication of the grant of the patent :
**30.11.88 Bulletin 88/48**

(84) Designated contracting states :
**BE DE FR GB IT NL SE**

(56) References cited :
**CH-A-   590 927**
**US-A- 4 206 282**

(73) Proprietor : **TERUMO KABUSHIKI KAISHA trading as TERUMO CORPORATION**
**44-1, 2-chome, Hatagaya Shibuya-Ku**
**Tokyo 151 (JP)**

(72) Inventor : **Shigematsu, Takashi**
**202, 3-4, 2-chome, Nagayama**
**Tama-shi Tokyo (JP)**

(74) Representative : **Henkel, Feiler, Hänzel & Partner**
**Möhlstrasse 37**
**D-8000 München 80 (DE)**

## Description

### Background of the Invention

This invention relates to a medium composition for examining biochemical properties of microorganisms. More particularly, it is concerned with a medium composition used for examining aerobic sugar assimilation of Gram-negative bacilli which are not capable of anaerobically assimilating glucose.

In order to apply an appropriate therapy for microbial infections it is important to identify pathogenic bacteria, conduct sensitivity tests and determine a drug active against the pathogenic bacteria. In the identification of pathogenic bacteria for the above purpose, a variety of examinations of biochemical properties is carried out, among which is the examination of the aerobic sugar assimilation for glucose-nonfermentative Gram-negative bacilli.

### Description of Prior Arts

As a medium for the above-mentioned examination has heretofore been employed a medium prepared by adding 10-20 g of sugar to a Hugh-Leifson oxidation fermentation medium having the following composition :

Composition of Hugh-Leifson OF Medium

|  | (g) |
|---|---|
| Peptone | 2.0 |
| NaCl | 5.0 |
| $K_2HPO_4$ | 0.3 |
| Agar | 2.0 |
| Bromthymol Blue | 0.08 |
| Water | 1 l |

The above medium is advantageous in that as it contains less peptone as compared with the medium for examining sugar assimilation of enterobacteriaceae, error of the judge caused by basic peptone degradates such as ammonia will be less. On the other hand, however, the medium is a gell medium in which agar is used so that diffusion of the acid formed in the medium requires a longer period of time, and formation of the acid is also slower. It takes one to three days until results of the examination are found.

In order to treat microbial infections at earlier stages it is necessary to carry out the identification of microorganisms as soon as possible, and development of the medium which enables identification of microorganisms in a shorter period of time has been desired.

In some cases, however, the judgment is made inevitably on the next day for the convenience of operation. In such a case, it is desirable to employ a medium with which strict fixation of cultivation period of time is not required, and extension of the cultivation period of time by certain reasons does not cause an excess reaction.

### Summary of the Invention

It is an object of the present invention to provide a medium composition for examining aerobic sugar assimilation of glucose-nonfermentative Gram-negative bacilli which enables the identification by cultivation for a shorter period of time as well as makes it possible to identify the microorganism correctly after cultivation for a longer period of time.

A further object of the invention is to provide a medium composition for the above described examination with which the color reaction is clear, and the judgment is easy.

According to the invention, there is provided a medium composition comprising one part by weight of peptone, 1-50 parts by weight of sodium chloride, 10-300 parts by weight of a sugar, 0.01-0.8 part by weight of an alkali metal dihydrogenphosphate, 0.12-2 parts by weight of a dialkali metal hydrogenphosphate and 0.05-2 parts by weight of a pH indicator. Further according to the invention, there is provided a medium composition which additionally contains 0.05-4 parts by weight of starch.

As the pH indicator in the medium of the invention is preferred Phenol Red.

### Detailed Description of the Invention

As set forth above, the medium of the invention comprises peptone, sodium chloride, a sugar, an alkali metal dihydrogenphosphate, a dialkali metal hydrogenphosphate and a pH indicator. Peptone is a proteinous nutrient. As it is employed in an amount less than half that in Hugh-Leifson OF medium, error in the examination caused by basic degradates such as ammonia is avoidable. The alkali metal dihydrogenphosphate and dialkali metal hydrogenphosphate act as pH-regulators. As the alkali metal is

2

used potassium or sodium. Combination of them is preferable. The sugar is the substrate and includes glucose, mannitol, xylose, lactose, maltose, trehalose, arabinose, adonitol, sucrose, fructose, galactose, mannose. Suitable pH indicators are Phenol Red, Bromthymol Blue, Bromcresol Purple, Cresol Red, Bromphenol Red, though any of pH indicators may be employed. Use of Phenol Red is especially suitable, because the color is clear and judgment whether it is positive or negative is easily made using a small amount of the examined material.

Proportions of the components in the medium of the invention described above are critical, and particularly the alkali metal dihydrogenphosphate and dialkali metal hydrogenphosphate are fixed to be in such an amount that said composition has a pH between 7.4 and 7.5 when dissolved in 1 l of water, and pH of the medium will be about 5 when the sugar substrate is converted to an organic acid.

When the above-described composition according to the invention additionally contains 0.05-0.4 g of starch, initial color of the medium is constant independent of the nature of the added sugars. Consequently, examinations for a variety of sugars can conveniently be carried out using a multiwell plate.

The culture medium composition of the invention is employed in liquid medium which is prepared by dissolving the components in amounts as defined above in 1 l of water. When simultaneous examinations for various sugars using a multiwell plate are to be carried out, a solution of the composition according to the invention dissolved in water is poured into a well in the plate for examination and dried to prepare a dry medium. In carrying out the examination, an aqueous suspension of the microorganism to be tested in a predetermined amount is poured into each of the well prepared as above, cultivation is carried out and a positive or negative sugar reaction is judged by the change in color of the culture liquor.

Whereas the medium according to the invention is used in exactly the same way as with other known media, cultivation period of time can be shortened to 3-5 hours since it is a liquid medium.

Cultivation for a longer period of time is also feasible. Therefore, the present medium is suitable for identification whenever it is done on the same day or on the next day. Judgment whether the reaction is positive or negative also is easily made because the color reaction is clear.

The medium composition of the invention will be described in more details below by means of examples.

## Examples

A medium composition of the invention with a composition as described in Table 1 was dissolved in 1 l of water. There were prepared the media I-VII for the identification of microorganisms. For comparison, control media I-VII were prepared by dissolving a medium composition with a composition as described in Table 1 (which is a medium obtained by adding a sugar to a Hugh-Leifson OF medium) in 1 l of water.

(See Table 1 page 4)

## Table 1

### Composition of the Medium

(g)

| Component \ Medium | Medium I | | Medium II | | Medium III | | Medium IV | | Medium V | | Medium VI | | Medium VII | |
|---|---|---|---|---|---|---|---|---|---|---|---|---|---|---|
| | A | B | A | B | A | B | A | B | A | B | A | B | A | B |
| Peptone | 0.1 | 2.0 | 0.5 | 2.0 | 0.5 | 2.0 | 0.5 | 2.0 | 0.5 | 2.0 | 1.0 | 2.0 | 0.5 | 2.0 |
| NaCℓ | 3.0 | 5.0 | 3.0 | 5.0 | 3.0 | 5.0 | 3.0 | 5.0 | 3.0 | 5.0 | 3.0 | 5.0 | 3.0 | 5.0 |
| $K_2HPO_4$ | – | 0.3 | – | 0.3 | – | 0.3 | – | 0.3 | – | 0.3 | – | 0.3 | – | 0.3 |
| $KH_2PO_4$ | 0.02 | – | 0.02 | – | 0.02 | – | 0.02 | – | 0.02 | – | 0.02 | – | 0.02 | – |
| $NA_2HPO_4$ | 0.18 | – | 0.18 | – | 0.18 | – | 0.18 | – | 0.18 | – | 0.18 | – | 0.18 | – |
| Glucose | 20 | 20 | – | – | 20 | 20 | – | – | – | – | 20 | 20 | – | – |
| Lactose | – | – | 20 | 20 | – | – | – | – | – | – | – | – | – | – |
| Mannitol | – | – | – | – | – | – | 20 | 20 | – | – | – | – | – | – |
| Maltose | – | – | – | – | – | – | – | – | 20 | 20 | – | – | – | – |
| Xylose | – | – | – | – | – | – | – | – | – | – | – | – | 20 | 20 |
| Agar | – | 2.0 | – | 2.0 | – | 2.0 | – | 2.0 | – | 2.0 | – | 2.0 | – | 2.0 |
| Phenol Red | 0.2 | – | 0.2 | – | 0.2 | – | 0.2 | – | 0.2 | – | 0.2 | – | 0.2 | – |
| Bromthymol Blue | – | 0.08 | – | 0.08 | – | 0.08 | – | 0.08 | – | 0.08 | – | 0.08 | – | 0.08 |

A The invention
B Control

0 206 277

The invention media I-VII and control media I-VII described above were poured respectively in an amount of 50 µl into a well of a multiwell plate. The media were dehumidified at 25 °C to dry medium. A culture of various microorganisms on an agar plate, obtained by cultivation at an optimum temperature for 18-24 hours, was suspended in distilled water to approximately $1.5 \times 10^9$ - $2.1 \times 10^9$ microorganisms per ml. Each well was then inoculated with 50 µl of the suspension and covered. The plate was incubated at 30 °C for a predetermined period of time, and sugar assimilation was judged by change in color of the culture liquor. Amount of the inoculated microorganism was $7.5 \times 10^7$ for the 5-hour culture medium and $1.5 \times 10^7$ for the 24-hour culture medium.

Results of the judgment are shown in Table 2. In the table, (+) or (—) indicates that the sugar assimilation was positive or negative, respectively.

(See Table 2 pages 6 and 7)

## Table 2

### Sugar Assimilation

| Medium Cultivation time (h) Microorganism | Medium I | | | | | Medium II | | | | | Medium III | | | | | Medium IV | | | | |
|---|---|---|---|---|---|---|---|---|---|---|---|---|---|---|---|---|---|---|---|---|
| | A | | B | | | A | | B | | | A | | B | | | A | | B | | |
| | 5 | 24 | 5 | 24 | 2* | 5 | 24 | 5 | 24 | 2* | 5 | 24 | 5 | 24 | 2* | 5 | 24 | 5 | 24 | 2* |
| Pseudomonas putida ATCC 12633 | + | + | − | + | + | − | − | − | − | − | + | + | − | + | + | − | − | − | − | − |
| Pseudomonas aeruginosa ATCC 10145 | + | + | + | + | + | − | − | − | − | − | + | + | + | + | + | + | + | − | + | + |
| Pseudomonas stutzeri ATCC 17588 | + | + | − | + | + | − | − | − | − | − | + | + | − | + | + | − | + | − | + | + |
| Pseudomonas cepacia ATCC 27515 | + | + | + | + | + | + | + | − | + | + | + | + | + | + | + | + | + | − | + | + |
| Agrobacterium radiobacter ATCC 4720 | + | + | − | + | + | + | + | + | + | + | + | + | − | + | + | + | + | − | + | + |
| Flavobacterium meningosepticum ATCC 27853 | + | + | − | + | + | + | + | − | + | + | + | + | − | + | + | + | + | + | + | + |

Table 2 (Continued)

Sugar Assimilation

| Medium / Cultivation time (h) / Microorganism | Medium V | | | | | Medium VI | | | | | Medium VII | | | | |
|---|---|---|---|---|---|---|---|---|---|---|---|---|---|---|---|
| | A | | B | | | A | | B' | | | A | | B | | |
| | 5 | 24 | 5 | 24 | 2* | 5 | 24 | 5 | 24 | 2* | 5 | 24 | 5 | 24 | 2* |
| Pseudomonas putida ATCC 12633 | − | − | − | − | − | + | + | + | + | + | + | + | − | + | + |
| Pseudomonas aeruginosa ATCC 10145 | − | − | − | − | − | + | + | + | + | + | + | + | − | + | + |
| Pseudomonas stutzeri ATCC 17588 | − | − | − | − | + | + | + | − | + | + | − | − | − | − | + |
| Pseudomonas cepacia ATCC 27515 | + | + | − | + | + | + | + | + | + | + | + | + | − | − | + |
| Agrobacterium radiobacter ATCC 4720 | + | + | − | + | + | + | + | − | + | + | + | + | − | + | + |
| Flavobacterium meningosepticum ATCC 27853 | + | + | − | + | + | + | + | − | + | + | − | − | − | − | − |

A The invention
B Control
* Week

As apparent from Table 2, use of the medium of the present invention in the examination for aerobic sugar assimilation of glucose-nonfermentative Gram-negative bacilli enables correct identification of the microorganism independent of the cultivation time. Therefore, the identification may be made either on the same day or on the next day for the convenience of examination operation. On the contrary, use of the control medium needs cultivation for at shortest 24 hours thereby making the identification on the same day infeasible. With the control medium I, for example, 5-hour cultivation of Pseudomonas putida ATCC 12633, Pseudomonas stutzeri ATCC 17588, Agrobacterium radiobacter ATCC 4720 or Flavobacterium meningosepticum ATCC 27853 produces a color reaction insufficient to make correct judgment. It is evident that correct judgment is impossible also with the 5-hour cultivation in the control media II-VII.

The 5-hour cultivation using the media of the invention enables the judgment as correct as that the 24-hour cultivation using the control media.

## Claims

1. A medium composition for the identification of microorganisms comprising one part by weight of peptone, 1-50 parts by weight of sodium chloride, 10-300 parts by weight of a sugar, 0.01-0.8 part by weight of an alkali metal dihydrogenphosphate, 0.12-2 parts by weight of a dialkali metal hydrogenphosphate and 0.05-2 parts by weight of a pH indicator.

2. A medium composition for the identification of microorganisms according to Claim 1 wherein the alkali metal dihydrogenphosphate is potassium dihydrogenphosphate and the dialkali metal hydrogenphosphate is disodium hydrogenphosphate.

3. A medium composition for the identification of microorganisms according to Claim 1 wherein the sugar is glucose, mannitol, xylose, lactose, maltose, trehalose, arabinose, adonitol, sucrose, fructose, galactose or mannose.

4. A medium composition for the identification of microorganisms according to any of Claims 1-3 wherein the pH indicator is Phenol Red.

5. A medium composition for the identification of microorganisms comprising one part by weight of peptone, 1-50 parts by weight of sodium chloride, 10-300 parts by weight of a sugar, 0.01-0.8 part by weight of an alkali metal dihydrogenphosphate, 0.12-2 parts by weight of a dialkali metal hydrogenphosphate, 0.05-4 parts by weight of starch and 0.05-2 parts by weight of a pH indicator.

## Patentansprüche

1. Mediumzusammensetzung für die Identifizierung von Mikroorganismen, enthaltend 1 Gew.-Teil Pepton, 1-50 Gew.Teil (e) Natriumchlorid, 10-300 Gew.-Teile eines Zuckers, 0,01-0,8 Gew.-Teil eines Alkalimetalldihydrogenphosphats, 0,12-2 Gew.-Teil (e) eines Dialkalimetallhydrogenphosphats und 0,05-2 Gew.-Teil (e) eines pH-Indikators.

2. Mediumzusammensetzung für die Identifizierung von Mikroorganismen nach Anspruch 1, dadurch gekennzeichnet, daß das Alkalimetalldihydrogenphosphat aus Kaliumdihydrogenphosphat und das Dialkalimetallhydrogenphosphat aus Dinatriumhydrogenphosphat bestehen.

3. Mediumzusammensetzung für die Identifizierung von Mikroorganismen nach Anspruch 1, dadurch gekennzeichnet, daß der Zucker aus Glucose, Mannit, Xylose, Lactose, Maltose, Trehalose, Arabinose, Adonit, Rohrzucker, Fructose, Galactose oder Mannose besteht.

4. Mediumzusammensetzung für die Identifizierung von Mikroorganismen nach einem der Ansprüche 1 bis 3, dadurch gekennzeichnet, daß der pH-Indikator aus Phenolrot besteht.

5. Mediumzusammensetzung für die Identifizierung von Mikroorganismen, enthaltend 1 Gew.-Teil Pepton, 1-50 Gew.-Teil (e) Natriumchlorid, 10-300 Gew.-Teile eines Zuckers, 0,01 bis 0,8 Gew.-Teil eines Alkalimetalldihydrogenphosphats, 0,12-2 Gew.-Teil (e) eines Dialkalimetallhydrogenphosphats, 0,05-4 Gew.-Teil (e) Stärke und 0,05-2 Gew.-Teil (e) eines pH-Indikators.

## Revendications

1. Une composition de milieu pour l'identification des micro-organismes, comprenant 1 partie en poids de peptone, 1 à 50 parties en poids de chlorure de sodium, 10 à 300 parties en poids d'un sucre, 0,01 à 0,8 parties en poids d'un dihydrogénophosphate de métal alcalin, 0,12 à 2 parties en poids d'un hydrogénophosphate de di (métal alcalin) et 0,05 à 2 parties en poids d'un indicateur de pH.

2. Une composition de milieu pour l'identification des micro-organismes selon la revendication 1, dans laquelle le dihydrogénophosphate de métal alcalin est le dihydrogénophosphate de potassium et l'hydrogénophosphate de di (métal alcalin) est l'hydrogénophosphate de disodium.

3. Une composition de milieu pour l'identification des micro-organismes selon la revendication 1, dans laquelle le sucre est le glucose, le mannitol, le xylose, le lactose, le maltose, le tréhalose, l'arabinose, l'adonitol, le saccharose, le fructose, le galactose ou le mannose.

4. Une composition de milieu pour l'identification des micro-organismes selon l'une quelconque des revendications 1 à 3, dans laquelle l'indicateur de pH est le rouge de phénol.

5. Une composition de milieu pour l'identification des micro-organismes, comprenant 1 partie en poids de peptone, 1 à 50 parties en poids de chlorure de sodium, 10 à 300 parties en poids d'un sucre, 0,01 à 0,8 parties en poids d'un dihydrogénophosphate de métal alcalin, 0,12 à 2 parties en poids d'un hydrogénophosphate de di (métal alcalin) 0,05 à 4 parties en poids d'amidon et 0,05 à 2 parties en poids d'un indicateur de pH.